Europäisches Patentamt

European Patent Office

Office européen des brevets

(11) Veröffentlichungsnummer: **0 396 857**
**A1**

(12) **EUROPÄISCHE PATENTANMELDUNG**

(21) Anmeldenummer: **90102853.0**

(22) Anmeldetag: **14.02.90**

(51) Int. Cl.⁵: **A61K 7/48, A61K 7/06**

(30) Priorität: **15.04.89 DE 3912477**

(43) Veröffentlichungstag der Anmeldung:
**14.11.90 Patentblatt 90/46**

(84) Benannte Vertragsstaaten:
**AT BE CH DE ES FR GB GR IT LI LU NL SE**

(71) Anmelder: **AZUCHEMIE, DR. MED. R. MÜLLER GMBH**
**Dieselstrasse 5**
**D-7016 Gerlingen(DE)**

(72) Erfinder: **Müller, Robert, Dr.**
**Herdweg 101**
**D-7000 Stuttgart(DE)**

(74) Vertreter: **Nöth, Heinz, Dipl.-Phys. et al**
**Patentanwälte Pfenning, Meinig & Partner**
**Mozartstrasse 17**
**D-8000 München 2(DE)**

(54) **Äusserlich anzuwendendes Präparat und seine Verwendung.**

(57) Ein äußerlich anzuwendendes Präparat mit einem Gehalt an Deanol bzw. dessen gebräuchlichen Salzen oder Estern sowie üblichen Formulierungshilfsstoffen, insbesondere für die Verwendung als Hauptpflegemittel zur Verbesserung der Hautstruktur und Hautelastizität, gegen vorzeitiges Altern und vorzeitige Faltenbildung der Haut, ferner als Sport-, Massage- und Hautfunktionsöl sowie Haarwuchsmittel und Mittel gegen Haarausfall.

EP 0 396 857 A1

## Äußerlich anzuwendendes Präparat und seine Verwendung

Die Erfindung betrifft ein auf der Haut anzuwendendes kosmetisches Präparat, das erfindungsgemäß gekennzeichnet ist durch einen Gehalt an 2-Dimethyl-aminoethanol (Deanol), insbesondere seiner Salze oder Ester.

Das Deanol kann dabei bevorzugt als Citrat, Hydrogencarbonat, Orotat, (RR)-hydrogentartrat, L-hydroglenglutamat, Aceglutamat, 4-acetamidobenzoat, Hydrogensuccinat usw. verwendet werden.

Die innerliche Anwendung von Deanol als Psychopharmakon bzw. Psychoenergetikum und als Geriatrikum ist seit Jahrzehnten medizinisch bekannt und üblich. Für Injektionslösungen wird bevorzugt die alkoholische Form, für Kapseln, Tabletten oder Dragees die Form von Deanol als Salz bzw. Ester organischer Säuren eingesetzt. Verschiedentlich handelt es sich auch um Kombinationspräparate, bei denen Deanol z. B. als Deanol-hydrogentartrat, Deanol-orotat, Deanol-citrat, Deanol-aceglutamat und anderen ester- oder salzartigen Verbindungen in Kombination mit Mineralstoffen, verschiedenen Vitaminen oder auch organischen Säuren, wie z. B. der in der Milch enthaltenen Orotsäure, und anderen Stoffen, wie Adenosin, Rutin und anderen, vorliegt. In der Geriatrie erfolgt die innerliche Anwendung zur Behandlung und Vorbeugung von altersbedingten Abnutzungserscheinungen.

Die Anwendung erfolgt vorwiegend aufgrund empirischer Erfahrungen, da der eigentliche biochemische Wirkungsmechanismus nicht bekannt ist und teils eine gewisse cholinergische, teils eine stimulierende Wirkung auf das zentrale Nervensystem angenommen wird.

Nachdem Deanol bzw. seine Verbindungen, wie Salze oder Ester, ausschließlich innerlich im Bereich der Geriatrie als Geriatrika oder als Psychopharmaka zur Anwendung kommen, hat sich nunmehr in völlig überraschender und unerwarteter Weise gezeigt, daß bei externer kosmetischer Anwendung auf der Haut ein eindrucksvoller günstiger Effekt auf deren Beschaffenheit, einschließlich dazugehörender Gewebsbezirke, resultiert.

Als äußerlich anzuwendende Zubereitung können kosmetische Cremes, Salben, Gele, Lotionen bzw. Liquida, Sport-, Massage- und Hautfunktionsöle, welche Deanol bzw. die beschriebenen Verbindungen enthalten, verwendet werden.

Hierbei kommt es unter der Anwendung zu einem günstigen Einfluß auf die Hautbeschaffenheit. Die Hautelastizität und die Hautstruktur werden verbessert und vorzeitiger Alterung und Faltenbildung vorgebeugt, so daß die Haut insgesamt frischer und jugendlicher erscheint. Bei lokaler Anwendung flüssiger Zubereitungsformen, z. B. als Haarwasser oder Haartinktur, kommt es zu einer Verminderung von androgenetisch bedingtem Haarausfall. Die Präparate werden üblicherweise aufgetragen und einmassiert.

Eine teilweise Erklärung für den günstigen Effekt auf die Hautbezirke findet sich inzwischen durch Untersuchungen, wonach z. B. in vitro durch Zugabe von Deanol die Proteinsynthese in Zellkulturen erhöht wird. Hierbei konnte gezeigt werden, daß z. B. durch Deanol-orotat eine Verlängerung der Lebensspanne mitotischer und postmitotischer menschlicher Hautfibroplasten induziert wird. Entscheidend kommt es hierbei auf die Anwesenheit und zelluläre Beeinflussung durch Deanol bzw. Deanol-orotat an.

Im folgenden werden Ausführungsbeispiele für verschiedene Zubereitungen angegeben.

| 1. 100 g Lotion enthalten: | |
|---|---|
| Polyoxyethylenstearylalkohol | 2,200 g |
| Polyoxyethylenfettsäureester | 3,80 g |
| Deanol-orotat | 0,65 g |
| Mittelkettige Triglyzeride | 4,00 g |
| Paraffinum perliquidum | 6,00 g |
| Propylenglykol | 4,00 g |
| Konservierungsmittel | q.s. |
| Duftstoffe | q.s. |
| gereinigtes Wasser | ad 100,00 g |

EP 0 396 857 A1

| 2. 100 g Sport- und Massageöl enthalten: | |
|---|---|
| Neutralöl | 60,00 g |
| Isopropylmyristat | 20,00 g |
| Duftstoffe | q.s. |
| Oxidationsinhibitor | q.s. |
| Deanol-orotat | 0,600 g |
| Paraffinöl | ad 100,00 g |

| 3. 100 g Haarwasser enthalten: | |
|---|---|
| Alkohol | 40,00 g |
| Duftstoffe | q.s. |
| Deanol-orotat | 1,0 g |
| gereinigtes Wasser | ad 100,0 g |

| 4. 100 g Salbe enthalten: | |
|---|---|
| Emulgierender Cetylstearylalkohol | 15,00 g |
| Ölsäureoleylester | 7,00 g |
| Mittelkettige Triglyzeride | 5,00 g |
| Propylenglykol | 4,00 g |
| Deanol-orotat | 1,00 g |
| Konservierungsmittel | q.s. |
| Duftstoffe | q.s. |
| gereinigtes Wasser | ad 100,00 g |

| 5. 100 g Creme enthalten: | |
|---|---|
| Polyoxyethylenfettsäureester | 5,00 g |
| Paraffinum perliquidum | 9,00 g |
| Mittelkettige Triglyzeride | 5,00 g |
| Stearinsäure | 4,00 g |
| Cetylalkohol | 2,00 g |
| Propylenglykol | 4,00 g |
| Deanol-orotat | 0,50 g |
| Konservierungsmittel | q.s. |
| Duftstoffe | q.s. |
| gereinigtes Wasser | ad 100,00 g |

Anstelle des Orotats können auch die oben als Beispiele angegebenen Salze und Ester des Deanols verwendet werden. Als Salze kommen bevorzugt die Na-, Ca- oder K-Salze zum Einsatz.

**Ansprüche**

1. Äußerlich anzuwendendes Präparat, gekennzeichnet durch einen Gehalt an 2-Dimethyl-aminoethanol sowie üblichen Formulierungsstoffen.

3

2. Präparat nach Anspruch 1, dadurch gekennzeichnet, daß das 2-Dimethyl-Aminoethanol als Salz oder Ester eingesetzt ist.

3. Präparat nach Anspruch 1 oder 2, dadurch gekennzeichnet, daß das 2-Dimethyl-aminoethanol als Hydrogencarbonat, Citrat, Orotat, Hydrogentartrat, Aceglutamat, Acetamidobenzoat oder Hydrogensuccinat eingesetzt ist.

4. Präparat nach einem der Ansprüche 1 bis 3, dadurch gekennzeichnet, daß es in Form von Salben, Cremes, Gelen, Lotionen, Ölen oder anderen Liquida sowie als Haarwässer und Haartinkturen vorliegt.

5. Verwendung eines Präparates nach einem der Ansprüche 1 bis 3 als pflegendes Kosmetikum.

6. Verwendung eines Präparates nach Anspruch 5 zur Verbesserung der Hautbeschaffenheit, der Hautstruktur und Hautelastizität, gegen vorzeitiges Altern und gegen vorzeitige Faltenbildung der Haut.

7. Verwendung eines Präparates nach einem der Ansprüche 1 bis 3 in flüssiger Form gegen Haarausfall und zur Förderung mangelnden Haarwuchses.

8. Verwendung eines Präparates nach einem der Ansprüche 1 bis 6 als Sport-, Massage- und Hautfunktionsöl.

# EUROPÄISCHER RECHERCHENBERICHT

**Europäisches Patentamt**

Nummer der Anmeldung

EP 90 10 2853

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-1 182 320 (R.W. PFIRRMANN) * Patentansprüche 1,2,3,15,16,17; Seite 2, Spalte 2, Zeilen 93-99 * --- | 1,2,3,1 ,4,6 | A 61 K 7/48 A 61 K 7/06 |
| A | DE-A-2 131 946 (MARTIN STORTO) * Patentanspruch 4 * --- | 1 | |
| A | "Martindale - The extra Pharmacopoeia", Auflage 28, 1982, Seite 1700, Verbindung 12.624-S, The Pharmaceutical Press, London, GB * Das ganze Dokument * ----- | 1,2,3 | |

**RECHERCHIERTE SACHGEBIETE (Int. Cl.5)**

A 61 K

Der vorliegende Recherchenbericht wurde für alle Patentansprüche erstellt

| Recherchenort | Abschlußdatum der Recherche | Prüfer |
|---|---|---|
| DEN HAAG | 21-08-1990 | SIERRA GONZALEZ M.T. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)